# EUROPÄISCHE PATENTSCHRIFT

(11) **EP 2 076 240 B1**
(45) Veröffentlichungstag und Bekanntmachung des Hinweises auf die Patenterteilung: **09.12.2009**
(21) Anmeldenummer: 07818323.3
(22) Anmeldetag: 21.09.2007
(51) Int. Cl.: A61K 8/44, A61Q 19/00

(54) **KOSMETISCHE ODER DERMATOLOGISCHE ZUBEREITUNGEN MIT EINEM GEHALT AN 2-ISOPROPYL-5- METHYL-CYCLOHEXANCARBONYL-D-ALANINMETHYLESTER UND EINEM ODER MEHREREN ANTI-ENTZÜNDLICHEN WIRKSTOFFEN**
COSMETIC OR DERMATOLOGICAL PREPARATIONS WITH A CONTENT OF 2-ISOPROPYL-5- METHYL-CYCLOHEXANECARBONYL-D-ALANINE METHYL ESTER ANND ONE OR MORE ANTI-INFLAMMATORY AGENTS
PRÉPARATIONS COSMÉTIQUES OU DERMATOLOGIQUES COMPRENANT DE L'ESTER MÉTHYLIQUE DE LA 2-ISOPROPYL-5- MÉTHYL-CYCLOHEXANECARBONYL-D-ALANINE ET UNE OU PLUSIEURS SUBSTANCES ACTIVES ANTI-INFLAMMATOIRES

(30) Priorität: 29.09.2006 DE 102006047153
(43) Veröffentlichungstag der Anmeldung: 08.07.2009
(73) Patentinhaber: Beiersdorf AG, 20253 Hamburg (DE)
(72) Erfinder: ECKERT, Julia, 22085 Hamburg (DE); KOLBE, Ludger, 21255 Dohren (DE); NEUFANG, Gitta, 20149 Hamburg (DE)
(86) Internationale Anmeldenummer: PCT/EP2007/008233
(87) Internationale Veröffentlichungsnummer: WO 2008/040460

(56) Entgegenhaltungen:
- EP-A- 1 639 993
- WO-A-02/30367
- WO-A-02/32381
- DE-A1- 2 205 255
- US-A1- 2006 171 912
- WATSON H R ET AL: "NEW COMPOUNDS WITH THE MENTHOL COOLING EFFECT" JOURNAL OF THE SOCIETY COSMETIC CHEMISTS, SOCIETY OF COSMETIC CHEMISTS,, US, Bd. 29, Nr. 4, 1978, Seiten 185-200, XP009045124 ISSN: 0037-9832

## Beschreibung

Die vorliegende Erfindung betrifft kosmetische oder dermatologische Zubereitungen mit einem Gehalt an 2-Isopropyl-5-Methyl-Cyclohexancarbonyl-D-Alaninmethylester und einem oder mehreren anti-entzündlichen Wirkstoffen.

Die vorliegende Erfindung betrifft in einer ganz besonderen Ausführungsform kosmetische und dermatologische Zubereitungen mit langanhaltender kühlender Wirkung, insbesondere hautpflegende kosmetische und dermatologische Zubereitungen.

Die vorliegende Erfindung betrifft in einer ganz besonderen Ausführungsform kosmetische und dermatologische Zubereitungen mit verminderter Hautreizung.

Die Kühlwirkung kosmetischer Zubereitungen beruht bisher auf zwei Grundprinzipien:

Einsatz von Komponenten die sich nach topischer Applikation gasförmig verflüchtigen und die hierfür erforderlich Energiemenge, die sog. Verdampfungsenthalpie, zu einem Großteil der Hautoberfläche entnehmen. Es werden daher in entsprechenden nicht okklusiven Kosmetika geeignete flüssige Komponenten eingesetzt. Als besonders geeignet hat sich hier Ethanol erwiesen, daneben zeigen Formulierungen mit hohem Wassergehalt ebenfalls eine deutliche Kühlwirkung.

Einsatz von sogenannten "Cooling Agents", die mit den Wärmerezeptoren der Haut in Wechselwirkung treten und somit ein Kälteempfinden auslösen, ohne eine meßbare physikalische Abkühlung zu generieren. Hierfür kommen insbesondere Menthol und diverse Mentholderivate (Frescolate, Physcool, Questice L, etc.) zum Einsatz. Insbesondere hohe Ethanolgehalte sowie Menthol und seine Derivate sind, neben dem irritativen Potential, insbesondere aufgrund ihres deutlichen Eigengeruchs für zahlreiche Einsatzzwecke unter olfaktorischen Gesichtspunkten nicht geeignet. Häufig genug bewirken solche Substanzen darüberhinaus gleichzeitig eine Durchblutungssteigerung, die im Gegenteil ein Wärmegefühl hervorruft.

Aufgabe der vorliegenden Erfindung war es, den Nachteilen des Standes der Technik abzuhelfen und Wirkstoffe und Zubereitungen, solche Wirkstoffe enthaltend, zur kosmetischen und dermatologischen Behandlung und/oder Prophylaxe erythematöser, entzündlicher, allergischer oder autoimmunreaktiver Erscheinungen, insbesondere Dermatosen, aber auch des Erscheinungsbildes des "Stingings" zur Verfügung zu stellen.

Ferner sollten solche Wirkstoffe bzw. Zubereitungen, solche Wirkstoffe enthaltend, zur Verfügung gestellt werden, welche zur Immunstimulation der Haut, dabei vorteilhaft auch zur Immunstimulation im Sinne der die Wundheilung fördernden Wirkung, verwendet werden können.

Der Begriff "Entzündung" ist ein relativ weitgefaßter und alter Begriff. Schon vor der Zeitenwende führte Aulus Celsus vier der fünf Kardinalzeichen der Entzündung ein: rubor, tumor, calor und dolor (Rötung, Schwellung, Hitze und Schmerz). Im 2. Jahrhundert definierte Galen von Pergamon das 5. Zeichen functio laesa (eingeschränkte Funktion). Insgesamt umfaßt die Entzündungsforschung 2000 Jahre, davon 200 Jahre auf zellulärem Level und 20 Jahre auf molekularem Level. Dabei wurde immer offensichtlicher das der Begriff uneinheitlich ist.

Entzündliche Erkrankungen sind gekennzeichnet durch Infiltrate von Entzündungszellen die jedoch sehr verschieden zusammengesetzt sein können. Psoriasis, eine entzündliche Hauterkankung, ist z. B. gekennzeichnet durch ein Infiltrat an oligoklonalen T-Zellen und polymorphkernigen Granulozyten in scharf umgrenzten entzündlichen Plaques. Die involvierte Haut des atopischen Ekzems hingegen ist gekennzeichnet durch infiltrierende T-Zellen gegen Umweltantigene und Eosinophilen Granulozyten. So vielfältig wie die entzündlichen Erscheinungsformen sind auch die Therapien mit anti-entzündlichen Substanzen.

Keinesfalls kann man davon ausgehen, das eine Substanz die bei der einen entzündlichen Erkrankung eine sehr gute Wirkung zeigt auch bei anderen Entzündungen genauso wirken wird. Deshalb wird in diesem Bereich intensiv geforscht, viele dieser meist chronischen Erkrankungen sind bis heute nicht zufriedenstellend therapierbar. Am nächsten kommen einer solchen allumfassenden Wirkung die Kortikosteroide, aber wegen der zum Teil gravierenden Nebenwirkungen kommen sie für eine kontinuierliche und längere Anwendung nicht in Frage. Für kosmetische Anwendungen sind Kortikosteroide aus diesem Grunde sogar ganz verboten, hier mussen andere Substanzen eingesetzt werden.

Hautberuhigende Kosmetika werden bei akuten Hautirritationen eingesetzt, diese sind von den oben beschriebenen (chronischen) Entzündungen abzugrenzen. Ursache für Irritationen können z. B. physikalische Reize wie UV-Strahlung oder Rasur sein. Insbesondere im Frühstadium und bei geringer Reizhöhe gibt es kein Infiltrat aus Entzündungszellen (T-Zellen, Makrophagen, Granulozyten,...), sondern die betroffenen Hautzellen (hauptsächlich Keratinozyten und Fibroblasten) produzieren selber eine Fülle von pro-entzündlichen Mediatoren. Diese Mediatoren aktivieren die Zellen, induzieren Abwehr- und Reparaturmechanismen und locken in der Folge dann Entzündungszellen an. Ziel der hautberuhigenden Wirkung von Kosmetika muß es also sein die negativen Folgen der Irritation zu verhindern, ohne die notwendigen Reparaturmechanismen zu blockieren. Die bekannten anti-entzündlichen Substanzen, die vor allem auf die infiltrierenden Zellen wirken sollen sind deshalb nur bedingt als Modellsubstanzen brauchbar.

Auch diesen Übelständen galt es abzuhelfen.

In der Literatur werden beispielsweise ionische Verbindungen, insbesondere Ammoniumsalze, als kühlende Agenzien beschrieben. Als kühlende Zubereitungen werden auch verbreitet isopropanolische Gele mit Campher- und Mentholzusatz angewandt.

Die Anwendung dieser Substanzen, namentlich auf gereizter Haut, ist jedenfalls problematisch. Darüber hinaus sind viele dieser Verbindungen schlecht wasserlöslich. Ihre Verwendung ist folglich auf wenige Kosmetika und Dermatika beschränkt.

DE-A-22 05 255 offenbart N-substituierte-p-Menthan-3-Carboxamide wie 2-Isopropyl-5-Methyl-Cyclohexancarbonyl-D-Alaninethylester und ihre Verwendung in kühlenden kosmetischen und dermatologischen Zubereitungen.

WO-A-02/32381 und WO-A-02/30367 offenbaren kosmetische Zubereitungen, die ein kühlende Wirkstoffe wie Menthol oder Carboxamide sowie anti-entzündlich wirkende Stoffe wie Panthenol, Allantoin und Vitamin E enthalten.

US-A1-2006/171912, EP-A-1 639 993 und H. Watson et al.: "New Compounds with the Menthol Cooling Effect" Journal of the Society of Cosmetic Chemists, Bd. 29, Nr. 4, 1978, Seiten 185-200, offenbaren die Verwendung von Alkyl Menthan Carboxamiden als Kühlungsmittel für Kosmetika.

Aufgabe der vorliegenden Erfindung war es also, pflegende kosmetische und medizinische Zubereitungen zur Verfügung zu stellen, die nicht die Nachteile des Standes der Technik haben, insbesondere solche, welche, auf der Haut oder Schleimhäuten angewandt, befeuchtend und/oder kühlend wirken.

Die DE 43 12 656 beschreibt die Verwendung kosmetisch oder pharmazeutisch unbedenklicher Substanzen mit positiver Lösungsenthalpie in kosmetischen oder medizinischen topischen Zubereitungen, **dadurch gekennzeichnet, dass** die Substanz oder die Substanzen in den Zubereitungen in einem weitgehend wasserfreien Medium vorliegen und/oder durch eine stoffliche Barriere von einem wasserhaltigen Medium abgeschirmt werden.

Obwohl dieses Vorgehen grundsätzlich zu kosmetisch befriedigenden Zubereitungen führen kann, sind diese jedoch galenisch äußerst aufwendig herzustellen.

Es war also die Aufgabe, der vorliegenden Erfindung, den Übelständen des Standes der Technik abzuhelfen und kühlende kosmetische oder dermatologische Zubereitungen zur Verfügung zu stellen, welche einfach herzustellen sind, keine Reizwirkung auf Haut oder Schleimhäute ausüben - beispielsweise unangenehmes Kribbeln oder Juckreiz - sowie bei bestimmungsgemäßer Anwendung angenehme Kühlung spenden.

Der Einsatz von Hautbefeuchtungsmittel - gerne verwendet werden mehrwertige Alkohole wie Glycerin - ist im allgemeinen erwünscht. Ein Nachteil von Zubereitungen des Standes der Technik ist aber, dass diese - sofern Polyolmengen eingesetzt werden, welche in bezug auf die Hautbefeuchtung eine gewisse Wirksamkeit zeigen - eine deutliche Klebrigkeit aufweisen und sich nur schlecht auf der Haut verteilen lassen.

Um die Klebrigkeit polyolhaltiger O/W-Emulsionen zu verringern und ein leichtes Hautgefühl zu erreichen (leicht verteilbar, kaum Rückstand, schnell einziehend), wird üblicherweise mit sehr dünnflüssigen Ölen gearbeitet. (d. h. mit Ölen mit einer Viskosität von 1 bis 15 mPa.s), die ferner eine sehr hohe Spreitfähigkeit (800 bis 1200 mm2/10 min) aufweisen.

Es war also Aufgabe der Erfindung, gute Hautbefeuchtung zu bewerkstelligen, ohne dass die kosmetische Anmutung der Zubereitungen darunter leidet.

Ferner war eine Aufgabe der vorliegenden Erfindung, Zubereitungen zur Verfügung zu stellen, welche den Zustand der Haut deutlich verbessern, insbesondere die Hautrauhigkeit vermindern.

Aufgabe war daher, all diesen den Nachteilen des Standes der Technik Abhilfe zu schaffen. Insbesondere sollten Produkte mit verringerter Klebrigkeit bzw. Schmierigkeit zur Verfügung gestellt werden. Produkte auf dem Gebiete der pflegenden Kosmetik, der dekorativen Kosmetik und der pharmakologischen Galenik sollten gleichermaßen von den geschilderten Nachteilen des Standes der Technik befreit werden.

Weiterhin war es eine Aufgabe der Erfindung, kosmetische Grundlagen für kosmetische Zubereitungen zu entwickeln, die sich durch gute Hautverträglichkeit auszeichnen.

Ferner war eine. Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

Erfindungsgemäß gelöst werden diese Aufgaben durch kosmetische oder dermatologische Zubereitungen mit einem Gehalt an 2-Isopropyl-5-Methyl-Cyclohexancarbonyl-D-Alaninmethylester und einem oder mehreren anti-entzündlichen Wirkstoffen.

Erfindungsgemäße Zubereitungen zeichnen sich durch hervorragende, angenehme Kühlwirkung und elegante kosmetische Anmutung aus. Sie sind nicht unangenehm Klebrig und stabil gegen physikalische Zersetzung wie beispielsweise Auf- oder Abrahmen ihrer Bestandteile. Sie zeichnen sich durch ein äußerst niedriges Reizpotential aus.

2-Isopropyl-5-Methyl-Cyclohexancarbonyl-D-Alaninmethylester zeichnet sich durch folgende Struktur aus:

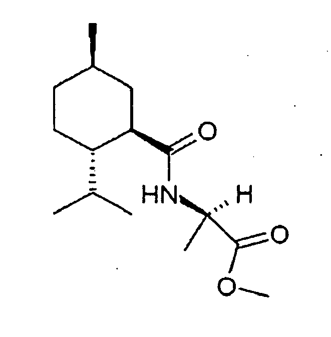

Die Synthese von 2-Isopropyl-5-Methyl-Cyclohexancarbonyl-D-Alaninmethylester [synonom: (R)-2-[((1R,2S,5R)-2-isopropyl-5-methyl-cyclohexancarbonyl)-amino]-propionsäuremethylester kann nach folgender Vorschrift erfolgen:

1,0 g D-Alaninmethylesterhydrochlorid (von Aldrich Chemical Co erhalten) wurde in 28 ml diethylether und 1 ml doppeltdestillierten Wassers aufgelöst und auf 0 °C gekühlt. Eine Spatelspitze des Katalysators Diaminopyrimidin wurde hinzugefügt. 1,62 ml p-Menthylchlorid wurden tropfenweise hinzugefügt, gefolgt von 2 ml Triethylamin. Flocken eines falrblosen Niederschlages bildeten sich in der Mischung, die über Nacht bei Raumtemperatur gerührt wurde. Der Niederschlag wurde in Ethylacetat gelöst, mit doppelt destilliertem Wasser gewaschen und über Natriumsulfat getrocknet. Die organische Phase wurde unter vermindertem Druck verdampft, wobei 2 g Endprodukt erhalten wurde, welches bei Raumtemperatur kristallisierte. Die erwartete Molekularmasse und Struktur wurden mit Massenspektrometer bzw. anhand des NMR-Spektrums bestätigt.

Vorteilhafte anti-entzündliche Wirkstoffe im Sinne der vorliegenden Erfindung sind beispielsweise Licochalcon A, Lignan, Chroman- oder Isoflavonoide, pentacyclische Triterpene (z.B. Ursolsäure, Glycyrrhetinsäure), Hamamelis Kamillenextrakte oder Bisabolol, Allantoin, Calendula Extrakte und/oder Panthenol.

Licochalcon A, zeichnet sich durch die folgende Strukturformel aus:

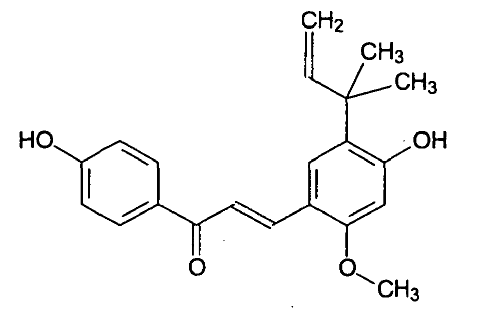

Es wird in Form wäßriger Extrakten aus der Wurzel der Pflanzenart *Glycyrrhiza inflata* gewonnen, die wie das in Europa offizinelle Süßholz *Glycyrrhiza glabra* der Gattung *Glycyrrhiza* angehört, die wiederum zur Pflanzenfamilie der Fabaceae (Erbsengewächse) gehört. Die Droge *Radix Glycyrrhizae inflatae,* d.h., die Wurzel der Pflanze, ist, beispielsweise in der fernöstlichen Medizin, gebräuchlich. Die Verwendung der Droge als Entzündungshemmer ist ebenfalls bekannt.

Bevorzugt enthalten kosmetische oder dermatologische Zubereitungen gemäß der Erfindung 0,001 - 10 Gew.-%, besonders bevorzugt 0,01 - 1 Gew.-%, an einem oder mehreren anti-entzündlichen Wirkstoffen, bezogen auf die Gesamtzusammensetzung der Zubereitungen.

Die kosmetischen oder dermatologischen Zubereitungen können erfindungsgemäß wie üblich zusammengesetzt sein und zur Behandlung, der Pflege und der Reinigung der Haut und/oder der Haare und als Schminkprodukt in der dekorativen Kosmetik dienen.

Sie enthalten bevorzugt 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an 2-Isopropyl-5-Methyl-Cyclohexancarbonyl-D-Alaninmethylester.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindugsgemäß vorteilhaft, Folsäure und/oder deren Derivate in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, Folsäure und/oder deren Derivate in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Siliconderivate.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Alle Mengenangaben, Anteile und Prozentanteile in den Beispielen sind, soweit nicht anders angegeben, Gewichtsprozente, auf das Gewicht und die Gesamtmenge bzw. auf das Gesamtgewicht der Zubereitungen bezogen.

| **Beispiel 1** (Referenzbeispiel) | **Gew.-%** |
|---|---|
| **Tagescreme** | |
| | |
| Glycerylstearat | 2,5 |
| Stearylalkohol | 2 |
| PEG-40-Stearat | 1 |
| Cetylalkohol | 2 |
| Sheabutter | 2 |
| Füllstoffe (SiO₂, Nylon) | 1 |
| Tapiokastärke | 1 |
| 2,4-Bis-{[4-(2-ethyl-hexyloxy)-2-hydroxy]-phenyl}-6-(4-methoxyphenyl)-1,3,5-triazin (Tinosorb® S) | 2 |
| Ethlyhexylmethoxycinnamat | 2 |
| Phenoxyethanol | 0,4 |
| C₁₂₋₁₅ Alkylbenzoat | 3 |
| p-Hydroxybenzoesäurealkylester (Paraben) | 0,4 |
| Aristoflex AVC (Ammonium Polyacryldimethyltaurid/Vinylformamid-copolymer) | 0,2 |
| Licochalkon A | 0,05 |
| 2-Isopropyl-5-Methyl-Cyclohexancarbonyl-D-Alaninethylester | 0,5 |
| Glycerin | 8 |
| EDTA | 0,2 |
| Parfüm | 0.3 |
| Natronlauge (pH 7 eingestellt) | q.s. |
| Wasser 100% | ad 100 |

| **Beispiel 2** (Referenzbeispiel) | **Gew.-%** |
|---|---|
| **Schutzcreme (Tagescreme)** | |
| Glycerylstearat | 3 |
| Stearylalkohol | 2 |
| PEG-40-Stearat | 1 |
| Sheabutter | 2 |
| 2,4-Bis-{[4-(2-ethyl-hexyloxy)-2-hydroxy]-phenyl}-6-(4-methoxyphenyl)-1,3,5-triazin (Tinosorb® S) | 2 |
| Ethlyhexylmethoxycinnamat | 5 |
| Phenoxyethanol | 0,4 |
| Dimethicon | 2 |
| Cyclomethicon | 4 |
| Titandioxid | 0,5 |
| Puderrohstoffe (Tapiocastärke, Nylon) | 3 |
| Tocopherylacetat | 1 |
| Retinylpalmitat | 0,3 |
| Panthenol | 2 |
| Carrageenan | 0,1 |
| Polyacrylsäure | 0,1 |
| 2-Isopropyl-5-Methyl-Cyclohexancarbonyl-D-Alaninethylester | 0,2 |
| Folsäure | 0,05 |
| Glycerin | 8 |
| Lignane | 0,2 |
| Parfüm | 0,3 |
| Natronlauge (pH 6.5 eingestellt) | q.s. |
| Wasser | ad 100 |

| **Beispiel 3** (Referenzbeispiel) | **Gew.-%** |
|---|---|
| **O/W-Emulsion** | |
| Glycerylstearatcitrat | 2 |
| Cetylalkohol | 3 |
| Sheabutter | 3 |
| Butylhydroxytoluol | 0,05 |
| 2,4-Bis-{[4-(2-ethyl-hexyloxy)-2-hydroxy]-phenyl}-6-(4-methoxyphenyl)-1,3,5-triazin (Tinosorb® S) | 2 |
| Ethylhexyltriazon | 2 |
| Squalan | 2 |
| Cyclomethicon | 5 |
| Dimethicon | 2 |
| Lauroyllysin | 2 |
| Carragenan | 0,15 |
| Carbomer | 0,1 |
| Kreatin | 0,2 |
| Süßholzextrakt | 0,03 |
| Glycerin | 8 |
| 2-Isopropyl-5-Methyl-Cyclohexancarbonyl-D-Alaninethylester | 0,2 |
| Parfüm | 0,3 |
| Natronlauge (pH 6.5 eingestellt) | q.s. |
| Wasser | ad 100 |

| **Beispiel 4** (Referenzbeispiel) | **Gew.-%** |
|---|---|
| **Pflegecreme** | |
| Hydrierte Kokosfettglyceride (Hydrogenated Coco Glycerides) | 2 |
| Stearylalkohol | 2 |
| Cetylalkohol | 2 |
| Cetearylglucosid | 2 |
| Ethlyhexylmethoxycinnamat | 3 |
| 2,4-Bis-{[4-(2-ethyl-hexyloxy)-2-hydroxy]-phenyl}-6-(4-methoxyphenyl)-1,3,5-triazin (Tinosorb® S) | 2 |
| Butylhydroxytoluol | 0,04 |
| Myristylmyristat | 1 |
| Macadamiaöl | 0,5 |
| Sheabutter | 2 |
| Cyclomethicon | 2 |
| Polyacrylsäure | 0,1 |
| Kreatin | 0,4 |
| Kamillenextrakt (Bisabolol) | 0,04 |
| Glycerin | 9 |
| EDTA | 0,1 |
| Parfum | 0,3 |
| Tocopherylacetat | 0,5 |
| 2-Isopropyl-5-Methyl-Cyclohexancarbonyl-D-Alaninethylester | 0,5 |
| Methylpropandiol | 2 |
| Natronlauge (pH 6.8) | q.s. |
| Wasser | ad 100 |

| **Beispiel 5** (Referenzbeispiel) | **Gew.-%** |
|---|---|
| **O/W-Emulsion** | |
| Caprylsäure/Caprinsäuretriglyceride | 3 |
| Sorbitanstearat | 1 |
| Stearylalkohol | 1 |
| Polyglyceryl-3 Methylglucosedistearat: | 3 |
| Cyclomethicon | 4 |
| 2,4-bis-[5-1(dimethylpropyl)benzoxazol-2-yl-(4-phenyl)-imino]-6-(2-ethylhexyl)-imino-1,3,5-triazin (CAS Nr. 288254-16-0) | 1 |
| Butyl Methoxydibenzoylmethane | 1 |
| Ethlyhexylmethoxycinnamat | 3 |
| Ethylhexyltriazon | 1 |
| Dimethicon | 2 |
| C₁₂₋₁₅ Alkylbenzoat | 2 |
| Dicaprylylether | 2 |
| Carrageenan | 0,1 |
| Vernetztes Alkylacrylat (Alkylacrylate Crosspolymer) | 0,1 |
| Kreatin | 0,4 |
| Bisabolol | 0,05 |
| Glycerin | 8 |
| EDTA | 0,2 |
| Licochalkon A | 0,02 |
| 2-Isopropyl-5-Methyl-Cyclohexancarbonyl-D-Alaninethylester | 0,4 |
| Citronensäure, Natriumsalz (pH 6.5) | q.s. |
| Wasser | ad 100 |

| **Beispiel 6** (Referenzbeispiel) | **Gew.-%** |
|---|---|
| **Lotion** | |
| Glycerylstearat | 2,5 |
| PEG-40-Stearat | 1 |
| Cetylalkohol | 2,5 |
| C₁₂₋₁₅ Alkylbenzoat | 2 |
| Hydrierte Kokosfettglyceride (Hydrogenated Coco Glycerides) | 2 |
| Octyldodecanol | 2 |
| Cyclomethicon | 2 |
| Bienenwachs | 1 |
| Ethylhexylmethoxycinnamat | 6 |
| Phenylbenzimidazolsulfonsäure | 2 |
| Natriumascorbylphosphat | 0,1 |
| Methylpropandiol | 2 |
| Diazolidinylharnstoff | 0,1 |
| Carrageenan | 0,1 |
| Carbomer | 0,1 |
| Glycerin | 7 |
| Panthenol | |
| EDTA | 0,2 |
| 2-Isopropyl-5-Methyl-Cyclohexancarbonyl-D-Alaninethylester | 0,5 |
| Natronlauge (pH 6.8) | q.s. |
| Wasser | ad 100 |

## Patentansprüche

1. Kosmetische oder dermatologische Zubereitungen mit einem Gehalt an 2-Isopropyl-5-Methyl-Cyclohexancarbonyl-D-Alaninmethylester und einem oder mehreren anti-entzündlichen Wirkstoffen.

2. Zubereitungen nach Anspruch 1, **dadurch gekennzeichnet, dass** das oder die anti-entzündlichen Wirkstoffe gewählt wird oder werden aus der Gruppe Licochalcon A (Süßholzextrakt), Lignan, pentacyclische Triterpene (z.B. Ursolsäure, Glycyrrhetinsäure), oder Isoflavonoide, Flavonoide, Bisabolol und Panthenol.

3. Zubereitungen nach einem der vorstehenden Ansprüche, **dadurch gekennzeichnet, dass** sie 0,001 Gew.-% bis 10 Gew.-%, bevorzugt 0,01 Gew.-% bis 1,0 Gew.-%, insbesondere 0,05 - 0,5 Gew.-%, bezogen auf das Gesamtgewicht der Zubereitungen, an 2-Isopropyl-5-Methyl-Cyclohexancarbonyl-D-Alaninmethylester enthalten.

4. Zubereitungen nach einem der vorstehenden Ansprüche, **dadurch gekennzeichnet, dass** sie 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an einem oder mehreren Antioxidantien enthalten.

## Claims

1. Cosmetic or dermatological preparations with a content of 2-isopropyl-5-methylcyclohexanecarbonyl-D-alanine methyl ester and one or more antiinflammatory active ingredients.

2. Preparations according to Claim 1, **characterized in that** the antiinflammatory active ingredient or ingredients is or are selected from the group consisting of licochalcone A (liquorice extract), lignan, pentacyclic triterpenes (e.g. ursolic acid, glycyrrhetic acid), or isoflavonoids, flavonoids, bisabolol and panthenol.

3. Preparations according to one of the preceding claims, **characterized in that** they comprise 0.001% by weight to 10% by weight, preferably 0.01% by weight to 1.0% by weight, in particular 0.05-0.5% by weight, based on the total weight of the preparations, of 2-isopropyl-5-methylcyclohexanecarbonyl-D-alanine methyl ester.

4. Preparations according to one of the preceding claims, **characterized in that** they comprise 0.001% by weight to 10% by weight, preferably 0.05% by weight to 5% by weight, in particular 0.1-2.0% by weight, based on the total weight of the preparations, of one or more antioxidants.

## Revendications

1. Préparations cosmétiques ou dermatologiques ayant une teneur en ester méthylique de 2-isopropyl-5-méthyl-cyclohexanecarbonyl-D-alanine et en une ou plusieurs substances actives anti-inflammatoires.

2. Préparations selon la revendication 1, **caractérisées en ce que** la ou les substances actives anti-inflammatoires est ou sont choisie(s) dans le groupe constitué par la licochalcone A (extrait de réglisse), le lignane, des triterpènes pentacycliques (par exemple l'acide ursolique, l'acide glycyrrhétinique) ou des isoflavonoïdes, des flavonoïdes, le bisabolol et le panthénol.

3. Préparations selon l'une quelconque des revendications précédentes, **caractérisées en ce qu'**elles contiennent de 0,001 % en poids à 10 % en poids, de préférence de 0,01 en poids à 1,0 % en poids, en particulier de 0,05 à 0,5 % en poids, par rapport au poids total des préparations, d'ester méthylique de 2-isopropyl-5-méthyl-cyclohexanecarbonyl-D-alanine.

4. Préparations selon l'une quelconque des revendications précédentes, **caractérisées en ce qu'**elles contiennent de 0,001 % en poids à 10 % en poids, de préférence de 0,05 % en poids à 5 % en poids, en particulier de 0,1 à 2,0 % en poids, par rapport au poids total des préparations, d'un ou plusieurs antioxydants.
